# EUROPEAN PATENT SPECIFICATION

(11) **EP 0 668 749 B1**
(45) Date of publication and mention of the grant of the patent: **30.01.2002**
(21) Application number: 94901415.3
(22) Date of filing: 10.11.1993
(51) Int. Cl.: A61B 17/60

(54) **SPINE OSTEOSYNTHESIS INSTRUMENTATION FOR AN ANTERIOR APPROACH**
WIRBELSÄULENOSTEOSYNTESE-INSTRUMENTARIUM FÜR EINE BEHANDLUNG VON VORN
INSTRUMENT D'OSTEOSYNTHESE VERTEBRALE PERMETTANT UNE APPROCHE ANTERIEURE

(30) Priority: 10.11.1992 FR 9213538
(43) Date of publication of application: 30.08.1995
(73) Proprietor: SOFAMOR, S.N.C., 62180 Rang-du-Fliers (FR)
(72) Inventor: HOPF, Christoph, D-6500 Mainz 1 (DE)
(74) Representative: Allman, Peter John
(86) International application number: US9310908
(87) International publication number: WO9410927

(56) References cited:
- US-A- 4 289 123
- US-A- 5 030 220
- US-A- 5 108 395
- US-A- 5 147 360

## Description

The present invention relates to a spinal osteosynthesis instrumentation for an anterior approach.

In the field of spinal instrumentation, instrumentation for a posterior approach has been widely explored and still remains an interesting solution in many pathological cases.

However, this technique has the drawback of not permitting direct access to the vertebral bodies, which often limits the use thereof and encourages an exploration of another approach, such as the anterior approach.

Although it is much more delicate and complex than the instrumentation for a posterior approach, the instrumentation for an anterior approach opens up a wider field of investigation. It is becoming necessary to investigate this field despite the fact that this approach requires considerable technical means and environment in the hospital and a specific training of very highly specialized orthopaedic surgery practitioners.

Depending on the vertebral level to be reached, several techniques of access by an anterior approach are at present practiced. They are:
Transpleural thoracotomy in the thoracic region,
Thoraco-phrenolumbotomy in the thoracolumbar region,
Lumbotomy in the lumbar region.

For each of these techniques, the approach on the left side of the vertebral column is preferred despite the proximity of the aorta, owing to the presence on the right side of the vena cava which is more fragile and less easy to move than the aorta.

However, the extension of the large vessels along the vertebral bodies has repercussions on the mounting of an instrumentation.

The technique by the anterior approach of the dorsolumbar spine is employed:
either for correcting a scoliosis and maintaining its correction,
or for treating a kyphosis with a large radius of curvature or an angular kyphosis.

Some surgeons are afraid of this technique; however, it is acknowledged to favour the treatment of certain pathologies. It is even indispensable in certain cases.

Whatever be the surgical schools of thought formed around spinal instrumentation, all surgeons acknowledge the following advantages of this technique:
Obtainment of an improved derotation of the vertical column and an improved correction of dorso-lumbar scolioses;
Obtainment of an improved instrumental stabilization, an improved correction of cyphoses, and an improved nerve decompression;
Direct viewing of the vertebral part to be treated in the anterior region and in the median region of the column;
Use of a relatively short length of instrumentation and fusion;
Higher fusion rate, approaching a value on the order of 95%;
Reduced medullary risks owing to a direct approach to the marrow and the necessity of distraction by a posterior action.

These advantages are of course not exhaustive and there could also be mentioned: an improved decompression, the non-destruction and therefore the preservation of the posterior stabilizing elements such as the muscles and ligaments, etc.

Resulting from the interest of the development of instrumentation for an anterior approach, many technical propositions have been made which may be resumed as follows:
Devices employing plates or the like fixed at the ends of the latter to the vertebrae located on each side of the vertebral region to be treated;
Devices employing rods, usually two rods, stiffened therebetween by transverse bars to which they are fixed thus forming a rectangular frame.

Each of the proposed structures has its specific advantages but also its drawbacks, with the result that the considered instrumentation is not or relatively rarely employed.

Two known examples may be mentioned:
French patent 2651992 (8912187, inventor J.M. LAURAIN) proposes the use of a rigid plate. Such a device corresponds to the specific requirements of fractures and tumors and of the degenerative field, but is hardly suitable for vertebral deviations.
French patent 2658413 (9001970, inventor J DUBOUSSET) proposes the use of two metal rods fixed along the vertebral column and interconnected transversely by two rigid bars, which imparts to the instrumentation a rectangular frame arrangement.

This solution is well suited to the problems of spinal deviations, but does not satisfy all the technological and surgical requirements.

In particular, the large number of parts making up the instrumentation implies a relatively long operating time and consequently this instrumentation, although effective is costly, which hinders its extension and marketing .

U.S. patent 4,289,123 (inventor H.K. Dunn) proposes an orthopedic appliance for use in an anterior approach. It describes the use of rocker arm brackets for attachment to the vertebra, with a pair of rocker arm brackets being joined across a damaged or diseased vertebra by a pair of threaded rods. However, with this system the connecting rods must be installed before attachment of the brackets to the vertebra, making installation of the appliance difficult.

In brief, the instrumentation for an anterior approach is very delicate and represents for the surgeons a high risk that technological innovation must attempt to reduce as far as possible.

Therefore, an object of the invention is to provide an instrumentation which permits overcoming these shortcomings and therefore treating, indifferently in a completely satisfactory manner, fractures and tumours as well as spinal deviations.

The invention provides an instrumentation spinal osteosynthesis instrumentation for an anterior approach, said instrumentation comprising in combination: two rigid rods at least two blocks for transversely interconnecting said blocks defining openings for respectively receiving one of said rods therein rods in such manner that said rods are axially slidable and revolvable about themselves in said blocks, means for clamping said rods against rotation and translation in said blocks and bone anchorage elements combined with said blocks, characterised in that said means for clamping said rods are slidably disposed within said blocks and bear against said rods.

Two blocks and the two associated rods thus make up a rectangular frame affording all of the required stability for restoring the axial continuity of the vertebral column, avoiding correction losses, and facilitating the consolidation of the bone graft.

According to an embodiment of the invention, each block has in a transverse direction, from an anterior part toward a posterior part of the vertebral bodies, a generally curved shape corresponding to the antero-lateral anatomy of the vertebral bodies, the anterior surface intended to be applied on a vertebral body being therefore concave while the exterior surface is convex.

According to other features:
The concave surface of the anterior part facing toward the vertebral body has a radius of curvature less than that of the exterior convex surface of the block;
Formed in each block are two parallel longitudinal recesses for receiving a respective rod, and a slide for clamping the rod, said slide being longitudinally slidable in the corresponding recess and becoming clamped on the rod at the end of its travel;
Each slide is provided with inclined lateral flanges adapted to be engaged in complementary lateral grooves in the recess, and said flanges cooperate with inclined clamping ramps provided in said grooves, or, inversely, the flanges are inclined and cooperate with clamping ramps provided in said recesses;
In a possible embodiment of the invention, the block is bent so as to have a substantially S-shaped cross-section the two halves of which constitute the anterior and posterior parts, the anterior part having a thickness less than that of the posterior part and being capable of being inserted in a recess provided for this purpose in the vertebral body.

Further features and advantages of the invention will be apparent from the following description with reference to the accompanying drawings which show several embodiments of the invention by way of non-limitative examples.

In the drawings:
Fig. 1 is a partly exploded perspective view of a first embodiment of the spinal osteosynthesis instrumentation for an anterior approach according to the invention.
Fig. 2 is a perspective view to a larger scale relative to Fig. 1 of a slide for closing the recess of the block shown in Fig. 1.
Fig. 3 is a perspective view of one of the blocks of the instrumentation shown in Fig. 1 to a smaller scale than in Fig. 2.
Fig. 4 is a partial top plan view of the instrumentation shown in Figs. 1 to 3.
Fig. 5 is a cross-sectional view taken on line 5-5 of Fig. 4.
Fig. 6 is a cross-sectional view taken on line 6-6 of Fig. 4.
Fig. 7 is an elevational view of the instrumentation shown in Figs. 4 to 6.
Fig. 8 is an elevational view of a second embodiment of the block of the instrumentation according to the invention.
Fig. 9 is a top plan view of the block shown in Fig. 8.
Fig. 10 is a half-elevational and half-sectional view taken on line 10-10 of Fig. 9.
Fig. 11 is a partial top plan view of an instrumentation according to the embodiment shown in Figs. 8 to 10.
Fig. 12 is an elevational view of the block and the screws shown in Fig. 11, the rods being shown in cross-section.
Fig. 13 is a top plan view of a vertebra, of a block according to the embodiment shown in Figs. 8 to 12 applied to said vertebra, and a distraction-compression tool whose end is engaged in a lateral opening in the block provided for this purpose.
Fig. 14 is an elevational view of an instrumentation according to that shown in Figs. 1 to 7, placed in position on a spinal segment having three vertebrae.

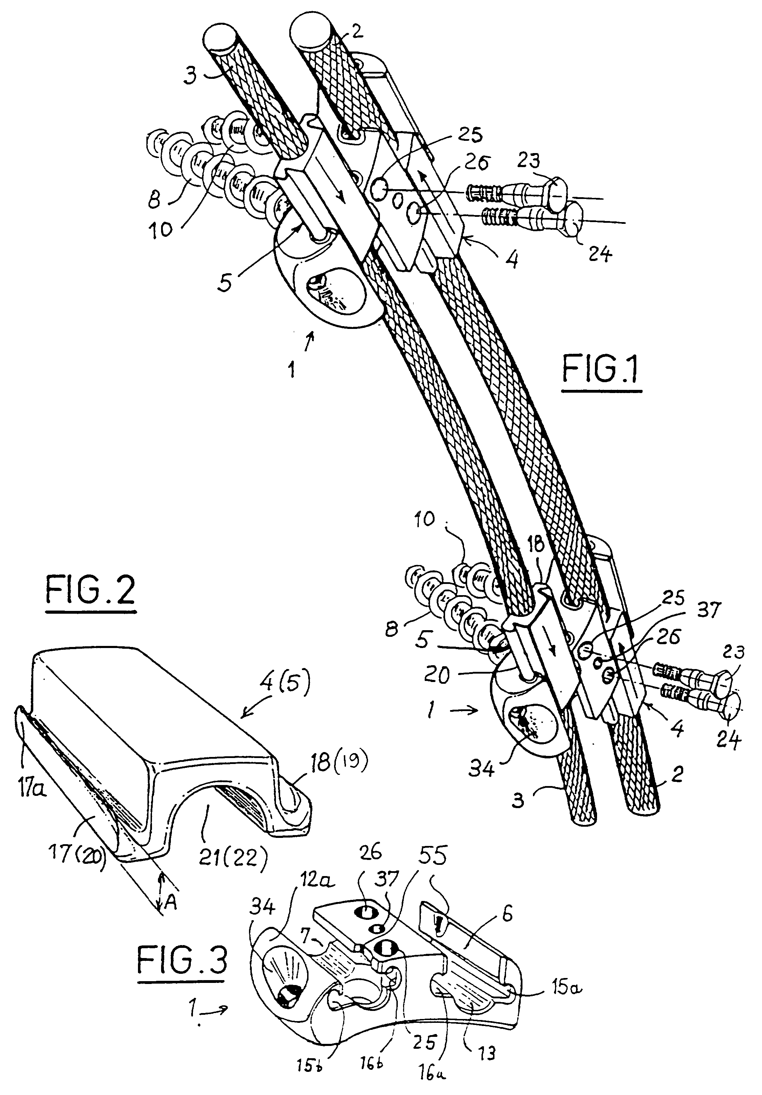

The spinal osteosynthesis instrumentation for an anterior approach shown in Figs. 1 to 7 comprises at least two blocks 1 adapted to transversely interconnect two rigid rods 2, 3 which are parallel to each other and axially slidable in said blocks. The instrumentation is shown in the vertical position viewed on the left side of the spine. The blocks 1 are provided with means for clamping the rods 2, 3 against rotation and translation and comprising essentially, for each block 1, two slides 4, 5 movable in translation in a direction parallel to the rods 2, 3 in corresponding recesses 6, 7 provided in the blocks 1.

Each block 1 is also provided with a pair of bone anchorage screws 8, 10 which may be placed in position by inserting them in corresponding bores 9, 11 provided in the bottom of the recesses 6 and 7. The bores 9, 11, and therefore the screws 8, 10, have suitable inclinations so that the two screws converge at a suitable angle, preferably on the order of 25°.

Advantageously, the screws 8, 10 have a conical head the taper of which corresponds to that of the bores 9, 11 so that, when they are placed in the bottom of the recesses 6, 7 or in the bottom of semi-cylindrical channels receiving the rods 2 and 3, they do not project and are blocked in axial abutment by the presence of the rod.

Cavities 55 provided in the surface of each recess (Fig. 3) facilitate the supporting of a boring tool (not shown) providing a prior bore for the bore 9, 11.

Each block 1 has, in the transverse direction from the anterior part toward the posterior part of the vertebral bodies on which they must be anchored by means of the screws 8, 10, a generally curved shape shown in particular in Figs. 5 to 7 and corresponding to the antero-lateral anatomy of the vertebral bodies. Thus, this outer surface has a substantially convex shape from the anterior surface S1 to the posterior surface S2, while the interior surface S3, adapted to be applied against the vertebral body, is concave.

Each block 1 has a substantially rectangular general shape of which the anterior region has a thickness less than that of the posterior region and the edges 12, 12a are rounded. Correspondingly, the radius of curvature of the anterior surface S1 is distinctly less than that of the posterior surface S2.

The upper surface (S2, S1) of each block is smooth owing to the fact that the surface of the slides 4, 5 are flush with the surrounding surface of the block, thereby avoiding any projection. The two recesses 6 and 7 extend in a direction parallel to each other and are each adapted to receive a portion of the respective rod 2, 3: the posterior rod 2 has a diameter distinctly larger than that of the rod 3 for a reason which will be explained hereinafter. The whole of the device must be disposed in most cases on the left side of the spine with the rod 2 in the posterior position.

Arranged in the bottom of each recess 6, 7 is a semi-cylindrical channel 13, 14 whose diameter corresponds to that of the respective rod 2, 3. Further, two lateral grooves 15a, 16a and 15b, 16b are formed in the sides of each recess 6, 7 in parallel relationship. These lateral grooves are adapted to receive corresponding lateral flanges 17, 18, 19, 20 of the respective slides 4 and 5. The flanges 17, 18, on one hand, and 19, 20, on the other, are arranged in a substantially different manner, as can be seen in Figs. 5 to 7, owing to the dissymmetry between the posterior and anterior parts of each block 1. Consequently, the same is true of their corresponding grooves (15a, 16a, 15b, 16b).

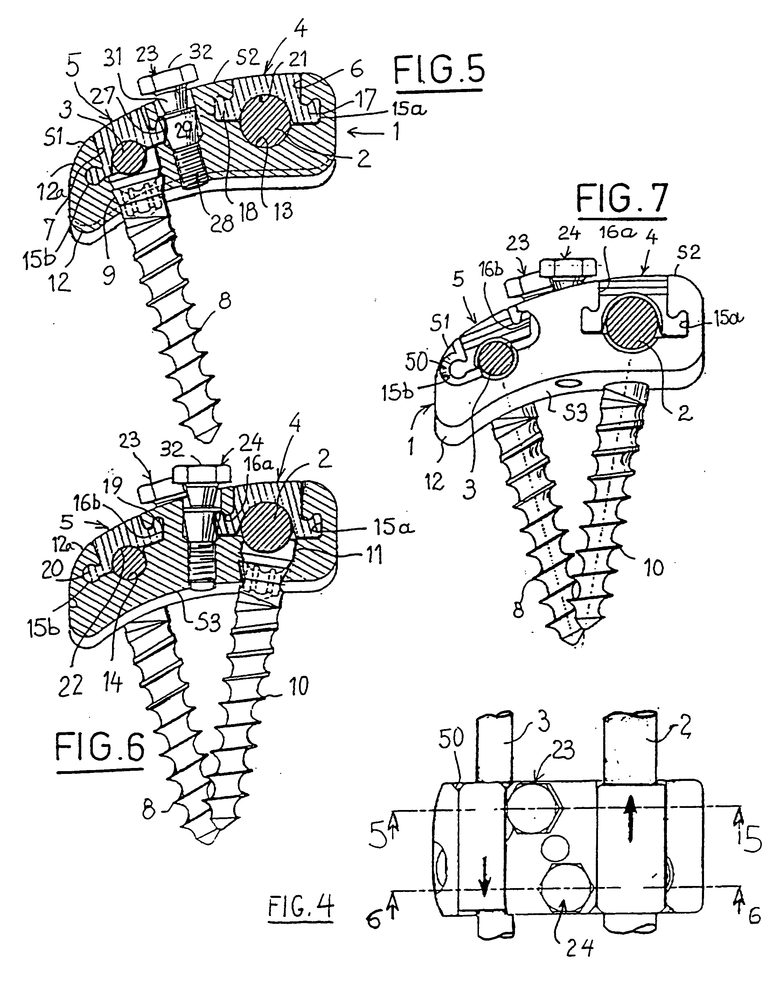

The flanges 17, 18 and 19, 20 are inclined at a suitable angle A (Fig. 2) on the bottom of the recesses 6, 7 in the same way as their respective lateral grooves 15a...16b which have for this purpose ramps of the same inclination A as the flanges 17...20. Thus the progressive insertion of each slide 4, 5 in the corresponding recess 6, 7 in the direction of the arrows (Figs. 1 and 4) results in a progressive gripping of the flanges 17, 18 and 19, 20 in their grooves 15a, 16a... and finally the clamping of the slides in the recesses 6, 7. The inclinations of the flanges (17...20) are inverted on the slides 4 and 5 so that the latter are inserted in the recesses 6, 7 in opposite directions indicated by the arrows (Figs. 1 and 4) to obtain a technical result which will be explained hereinafter. The angle of inclination A of the flanges 17, 18 and 19, 20 and of the ramps may be between about 2 and 6°.

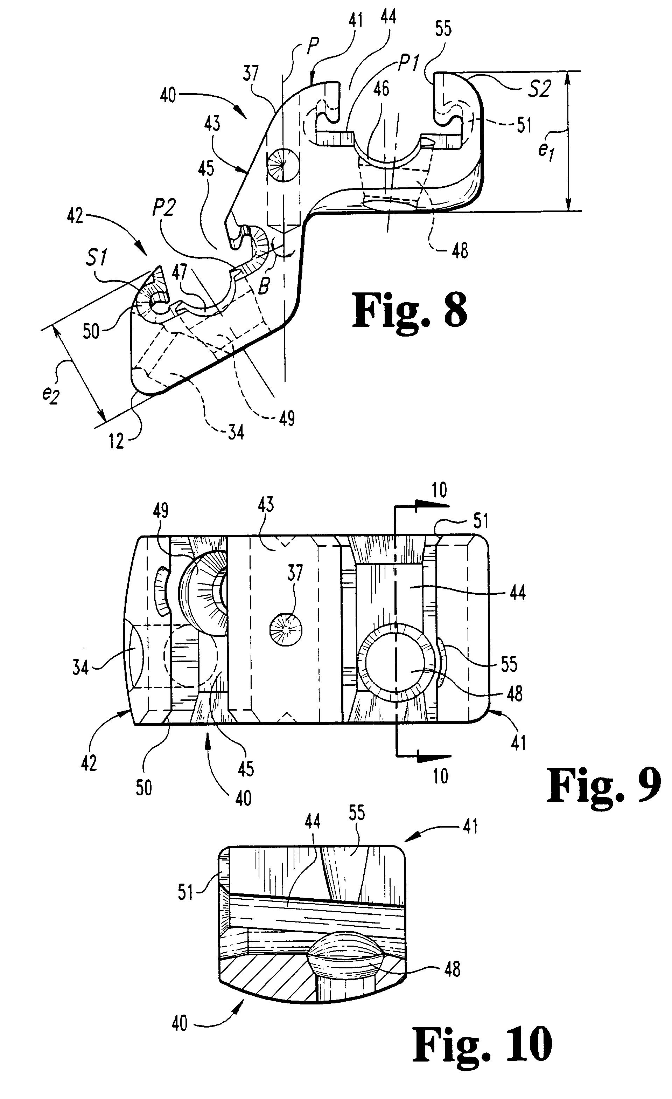

The entry ends of the flanges (19...20) are advantageously machined in such manner as to have chamfers or rounded edges, such as 17a (Fig. 2). Correspondingly, the entry edges of the recesses 6, 7 also have chamfers not, shown in Figs. 1 to 7 but shown in Figs. 8 to 12 (50, 51). In cooperation with the chamfers 17a, these chamfers facilitate the insertion of the slides 4, 5 in their recesses 6, 7.

It should be noted that the semi-cylindrical channels 13, 14 are of course completed by corresponding channels 21, 22 provided in the slides 4, 5 (Figs. 5 to 7).

Each block 1 can include complementary clamping means for each slide 4, 5. In the illustrated embodiment, these clamping means comprise, for each slide 4, 5, a respective screw 23, 24 which can be inserted in an opening 25, 26 provided in the central part of the block 1 between the recesses 6, 7. The opening 25 for the screw 23 communicates through a lateral passage 27 (Fig. 5) with the adjacent groove 16b of the lateral flange 19 of the slide 5. Likewise, the opening 27 of the passage between the opening 26 for the screw 24 and the groove 16a of the flange 18 of the slide 4 is shown in Fig. 5. Each screw 23, 24 has, from one end to the other, a screw threaded bone anchorage stem 28, a conical portion 29 which tapers toward the screw threaded stem 28, a fracture cone 31 which tapers toward the conical portion 29, and lastly a screwing head 32.

The end of the screw threaded portion 28 is rounded and is substantially flush with the interior surface S3 of the block 1. This screw threaded portion 28 is so dimensioned, as is the conical portion 29, that the latter bears against the adjacent flange 18, 19 of the respective slide 4, 5 by extending through the opening of the passage 27 (Figs. 5 and 6).

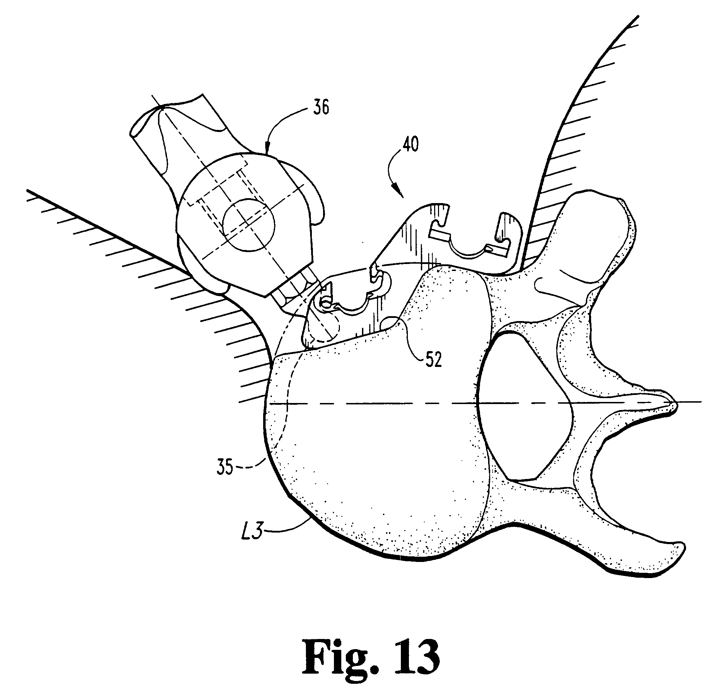

An aperture 34 is provided in the anterior part of each block in the surface S1 between the recess 7 and the rounded edge 12. This aperture 34 is adapted to receive the rounded head 35 of a distraction-compression tool 36 (Fig. 13) which is known per se and does not require description.

The implantation of the block 1 or "clip-block" requires the use of specific ancillary devices:
Ancillary device for mounting the slides 4 and 5: on each side of these slides, i.e. on the edges of their free ends, there is provided a notch which permits an effective and firm engagement of the ancillary device;
Ancillary device for mounting the block 1: an opening 37 provided in the central part of the block 1 (Fig. 1) facilitates the engagement;
Distraction ancillary device: the bore 34 in the anterior part of the blocks 1 permits the engagement of the distraction ancillary device 36 on either one of the clip-blocks 1.

The posterior rod 2 may have a diameter of 4 to 7 mm while the anterior rod 3 has a smaller diameter of 3 to 6 mm. This difference between the diameters is explained by the fact that it is on the posterior rod 2 that the main forces are exerted, in particular those due to derotation. On the other hand, the anterior rod 3 is subjected to smaller mechanical forces and may consequently have a smaller diameter. Further, this anterior rod 3 is the closest to the large blood vessels so that its reduced diameter increases the surgical safety of the instrumentation. However, this difference in diameter is not obligatory and the two rods 2 and 3 may possibly have the same diameter.

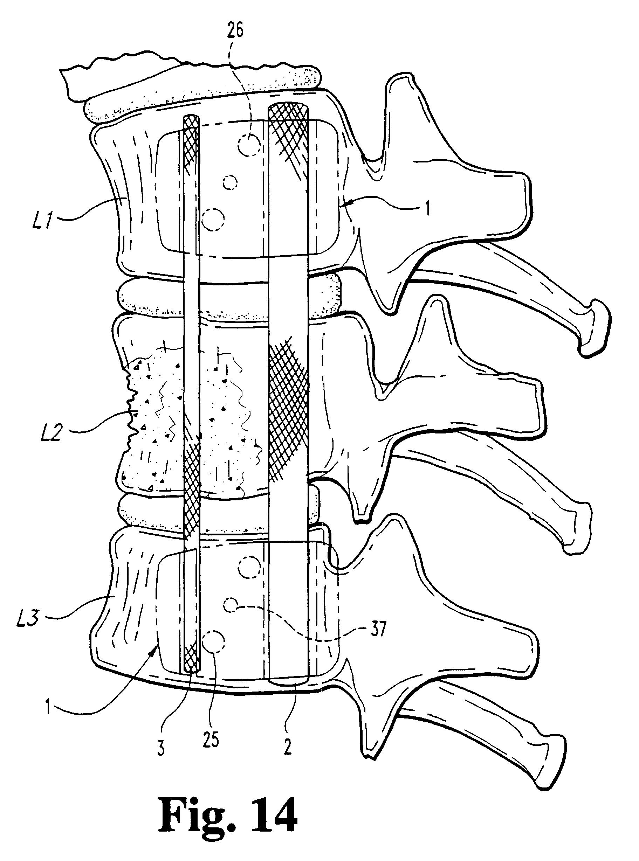

Fig. 14 shows an embodiment of the instrumentation according to the invention constituting a short arrangement extending along a vertebral segment of three vertebrae L3, L2, L1. The sequence of the mounting of the component parts of this instrumentation is the following:
A block 1 is placed on and then secured to the antero-lateral side of the body of the vertebra L3 by means of vertebral screws 8, 10. Another block 1 is also placed in position and then secured in the same way to the body of the vertebra L1, the vertebra L2 being the vertebra to be treated.

A posterior rod 2, for example having a diameter of 6 mm, is placed in the posterior recesses 6 in the upper block and lower block. By means of a suitable ancillary device, the posterior slides 4 corresponding to the recesses 6 are installed on their respective inclined ramps 6a, 7a and are placed in contact with the upper side of the rod 2. This rod is thus trapped in the recesses 6 of the blocks but remains movable in translation and rotation under the action of outside forces. This is why the position of the slides on their clamping ramps, or intermediate position, must be such as to permit acting in complete safety on the rod without allowing the latter to escape from its recess. The slides are consequently not pushed to the end of their inclined ramps.

Such an intermediate position of the slides enables the surgeon to effect if necessary a derotation action on the vertebral column by revolving the posterior rod 2 about itself. Corresponding to this derotation is a new position of the rod 2 relative to the connecting recesses 6, this position being automatically obtained in accordance with the nature of the change brought about in the vertebral column by the derotation effect.

Then an anterior rod 3, for example having a diameter of 4 mm, of the same length as the posterior rod 2 in the embodiment shown in Fig. 14 (no slides are shown), is inserted in the anterior recesses 7 of the upper block and lower block. As for the rod 2 and the slides 4 of the recesses 6, the anterior slides 5 are placed on their respective inclined ramps in such manner as to maintain the rod 3 in a trapped position but sufficiently free to move in particular in translation. Their gripping action round the rod is therefore not complete as they are not pushed to the end of their inclined ramps.

Thus, depending on the nature of the pathology to be treated, an intervention involving distraction or compression of the vertebrae relative to one another may be performed by the surgeon by means of the ancillary device 36 or a like ancillary device the studs 35 of which are inserted in the bores 34 located in the anterior parts of the two blocks.

As soon as the desired distraction or compression is obtained, all the slides 4, 5 of the blocks are pushed right to the ends of their respective ramps and thus exert on the rods 2, 3 a progressively increasing gripping pressure which firmly clamps the rods in their respective recesses 6, 7 against rotation and translation.

As soon as the slides 4, 5 are fully clamped, the installation of the instrumentation is finished. Owing to the constitution of the blocks according to the invention, the rectangulation or framing effect of the appliance, similar to that achieved by a plate, is obtained without the addition of complementary parts such as transverse connection rods.

Lastly, in order to afford an additional safety in the clamping of the rods and slides in their recesses, the screws 23, 24 are inserted in the apertures 25, 26, tightened and then broken off in the region of their fracture cone provided for this purpose.

As concerns the constitution of the instrumentation according to the invention, it is important to notice the reliability of the device, in particular in the anchorage of the blocks 1 to the vertebrae. The anchorage screws 8, 10 are indeed intentionally placed at the bottom of the recesses and advantageously possess a conical head (Figs. 5 and 6) corresponding to the taper of the bores 9, 11 so that the head does not assume a projecting position in the semi-cylindrical channels 13, 14 receiving the rods 2, 3.

When the instrumentation is definitively installed, i.e. after completely closing the slides 4, 5, the anchorage screws 8, 10 are also clamped and can in no way, under the multiple effects of the movements of the body of the patient, become detached from the vertebral column in which they are inserted. Their axial displacement is rendered impossible so that, in the event of a possible fracture in any region of its length, the two screw portions are held fast and nonetheless maintain the block.

Note also that the slides of each rod are inserted in the same axial direction, but for each of the rods the direction of insertion of the slides is inverted, as shown by the arrows in Figs. 1, 4 and 11 engraved on the upper surface of the slides 4, 5. In this way, as the inclined ramps of the recesses 6, 7 are arranged to permit such an inverted insertion of the slides of each block 1, after the slides are clamped in position, all the forces exerted by the multiple movements of the patient on one of the rods which tend to untighten the assembly are taken by the other rod in the opposite direction for exerting gripping forces. They are in this way neutralized, this arrangement therefore considerably increasing the reliability of the whole of the instrumentation.

The instrumentation according to the invention has still further technical advantages:

First of all, the rectangular structure obtained by the assembly of two clip-blocks 1 with their slides 4, 5 and the two rods 2, 3, constitutes a device for the fixation and the rectangulation of the assembly providing an improved stabilization. In instrumentations of the prior art having rods, it was necessary for the two rods to be definitively fixed for placing in position a transverse connection device (rectangulation). Owing to the present invention, the rectangulation of the device is automatically obtained by the mounting of the anterior consolidation rod 3.

Strength for resisting physiological loads over a period of time, avoiding an excessively long fusion, permitting a rapid mobilization of the patient with no external immobilization, and avoiding an additional fixation by the posterior approach requiring a second surgical intervention.

Possibility of an instrumental correction in the three dimensions so as to restore the spinal profile (by a relative compression or distraction of the vertebrae), possibility of a complete correction of deviations, possibility of a complete correction of the vertebral rotation and of the gibbosity of the column for the reequilibrium of the latter.

Small volume not only as concerns thickness so as to avoid contact with the large vessels, but also as concerns the amount of metal employed. For example, this thickness should not exceed 11 mm in the posterior third of the vertebral body and 9 mm in the middle third. This avoids any interference with the consolidation of the bone graft.

Simplicity of positioning by the surgeon, owing to the small number of component parts making up the instrumentation. This reduces the number of ancillary devices required and consequently the overall cost of the instrumentation.

Facility and rapidity of implantation by the surgeon who, for the reasons mentioned before, must as far as possible reduce any risk of neurological injury.

Flexibility of utilization permitting the instrumentation to be not only universal in its spinal application but also to be adapted to all levels of the vertebral column, in particular from T4 to L5, with implants of different sizes and different shapes.

Considerable safety of utilization owing to the fact, in addition to the foregoing features, that each anchorage screw 8, 10, once inserted in its bore 9, rests therein in a completely trapped manner. Indeed, the cylindrical rod 2, 3 is clamped on the head of the screw 8, 10 by the corresponding slide 5, 4 after the complete closure of the latter. Consequently, the screw is completely blocked in the axial direction and, if a transverse fracture of its screw threaded portion occurs, its consequences on the patient would be slight if not practically nil.

The force exerted for completely closing the slides 4, 5 is sufficiently high to prevent the accidental opening of the latter owing to the action of each longitudinal flange (17...20) and its chamfered end 17a which permits a privileged insertion in one direction rather than in the other (see Figs. 1 and 4). This is the reason for the provision of arrows on the surface of the slides 4 and 5 which indicate the direction of insertion of each slide. This arrangement is made necessary by the slope of the sliding and gripping longitudinal flanges.

The upper surface of each clip-block 1 is completely smooth, in particular in its most rounded lateral part S1 corresponding to the anterior positioning on the vertebrae. The upper surface of the slides 4, 5 which is also smooth is flush with the surfaces S1, S2 of the lateral parts and of the central part of the block 1, so that no particular element projects. This is a notable advantage of the clip-block provided by the invention.

The screws 23, 24 clamping the slides 4 and 5 afford additional safety to the instrumentation by the tightening of their conical portions 29 on the flanges 18 and 19 of the slides 4, 5. It concerns an additional safety means with respect to the clamping of the slides 4, 5 on the rods C-D 2 and 3, since the clamping is in any case assured by the clamping of the slides on the rods. The screws 23, 24 therefore only have an additional subsidiary effect. In order to avoid projecting from the surface of the block 1, each screw 23, 24 is broken off in its upper part after tightening on the block, with the aid of the fracture conical portion 31. This fracture therefore occurs at the junction between the fracture conical portions 31 and the tightening conical portions 29, in the weakest region. Thus each screw 23, 24 is practically hidden in the block 1 since the fracture plane is approximately at a distance of 2 to 3 mm from the surface S2 of the block 1.

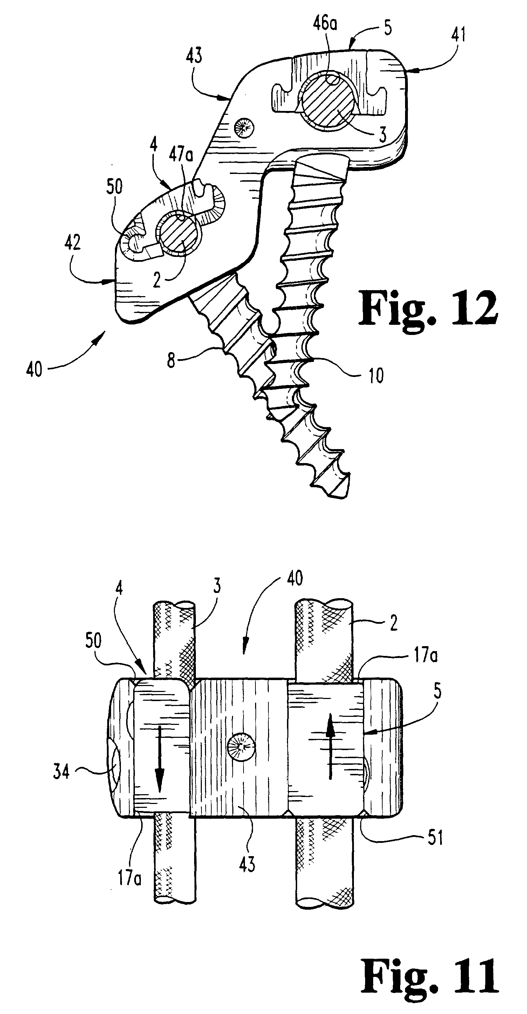

In the second embodiment of the invention shown in Figs. 8 to 13, each block 40 has a generally bent shape so as to have an approximately S-shaped cross-section. The two halves 41 and 42 of the block 40 respectively constitute its posterior and anterior parts, the posterior part 41 having a thickness e1 greater than the thickness e2 of the anterior part 42. The two parts 41, 42 are interconnected by a central part 43 which extends in a general direction P substantially perpendicular to the plane P1 of the bottom of the recess 44 of the-posterior part 41. On the other hand, the general direction P in which the central part 43 extends makes an angle B of less than 90° with the plane P2 of one of the sides of the bottom of the recess 45 of the anterior part 42. As in the blocks 1, semi-cylindrical channels 46, 47 are provided in the bottom of the respective recesses 44, 45 and complementary channels 46a, 47a in the slides 4, 5, the diameters of said channels corresponding to those of the rods 2 and 3. Bores 43, 49 are provided in the bottom of the channels 46, 47 for receiving anchorage screws 10 and 8 respectively. Note that in Figs. 8 and 9 the entry chamfers 50, 51 of the recesses 44 and 45 facilitate the insertion of the corresponding slides 4, 5. Further, the arrangement of the block 40 is similar to that of the block 1, the same reference numerals being used for the corresponding parts in both cases.

When the component parts of this instrumentation have been assembled (Figs. 11 and 12), the anchorage screws 10 and 8 respectively extend through the posterior part 41 and anterior part 42 and make an angle therebetween on the order of 35 to 40°. The anterior part 42 is then placed in a vertebral recess 52 (Fig. 13) produced by the surgeon and opening onto the anterior part of the vertebra, for example L3 or L1. Thus the anterior part 42 of the clip-block 40 is inserted in the vertebral region. The angle between the posterior part 41 and anterior part 42 (corresponding to the angle between the two planes P1 and P2) affords additional safety to the surgeon bearing in mind the proximity of the large vessels in the anterior approach.

The edge 12 of the anterior part S1, which is slightly rounded in the transverse direction as already mentioned, may have a radius of curvature of about 15 mm. This specific shape corresponds to the mean estimation of the value of the concavity of the vertebra in the region of the implantation of the block 1, 40 on the latter. This disposition contributes to the obtainment of the desired stability of the whole of the instrumentation owing to the fact that there is in this way an improved distribution of the forces exerted by the concerned faces in contact.

As explained before, the directions of insertion of the slides 4 and 5 are opposite so that over a period of time, when the patient resumes his activity, no loosening of the slides can occur. Indeed, the forces which tend to loosen the slide or slides 4 clamping the posterior rod 2 (which is used for the derotation), can be opposed or balanced by the forces tending to loosen the slide or slides 5 clamping the anterior rod 3. Experience has shown that it is desirable to clamp the slides 4 on the posterior rod 2 in the upward direction while the clamping of the other rod 3 then occurs in the downward direction.

Such an assembly has an excellent mechanical behaviour. In strength tests with a static and dynamic testing machine, employing the maximum forces normally produced by the human body, it was not possible to detect a loss of connection.

It must be understood that the scope of the invention is not intended to be limited to the two described embodiments since many alternative embodiments may be envisaged. For vertebral segments longer than that illustrated in Fig. 14, the instrumentation may have the required number of clip-blocks 1, for example three or four.

Likewise, it could be necessary that, as opposed to the embodiment shown in Fig. 14, the two rods 2 and 3 be exceptionally different, for example that the posterior rod 2 extend beyond the anterior rod 3. In this case, the rods may be fixed as previously described, and the extending portion of the rod may be fixed by at least one clip-block according to the invention but defining a single recess 6 or 7. This block is then fixed so as to maintain the additional length of this rod, the recess for receiving the other rod being unnecessary.

The additional means for clamping the slides 4, 5 constituted by the pairs of screws 23, 24 may comprise only a single screw per block 1, which may be inserted in a central aperture in the block. This aperture and the screw then have a diameter which is sufficient to ensure that the aperture communicates through lateral passages with the adjacent grooves (16a, 16b) of the lateral flanges 18, 19 of the slides 4, 5. The screw is provided with a conical portion 29 positioned to engage in said lateral passages and come to bear against the-flanges of the slides so as to reinforce their clamping in their recesses.

In this case, additionally, the aperture 34 may be advantageously eliminated, it being possible to carry out the distraction-compression operation by inserting the head of the ancillary device 36 in the central aperture of the screw.

## Claims

1. Spinal osteosynthesis instrumentation for an anterior approach, said instrumentation comprising in combination: two rigid rods (2,3), at least two blocks (1) for transversely interconnecting said rods (2,3), said blocks (1) defining openings for respectively receiving one of said rods therein in such manner that said rods (2,3) are axially slidable and revolvable about themselves in said blocks (1), means (4,5) for clamping said rods (2,3) against rotation and translation in said blocks (1), and bone anchorage elements (8,10) combined with said blocks (1) **characterized in that** said means (4,5) for clamping said rods (2,3) are slidably disposed within said blocks (1) and bear against said rods.

2. Instrumentation according to claim 1, wherein said blocks (1) are adapted to receive said rods (2,3) in a parallel arrangement.

3. Instrumentation according to claim 1, wherein each block (1) has an anterior part and a posterior part and a generally curved shape in a transverse direction from said anterior part to said posterior part corresponding to the antero-lateral anatomy of vertebral bodies, said block (1) defining an interior surface (S3) for application on a vertebral body and which is therefore concave, and a convex exterior surface (S1,S2).

4. Instrumentation according to claim 3, wherein said anterior part has a thickness less than the thickness of said posterior part and has a rounded edge.

5. Instrumentation according to any preceding claim, wherein each block (1) defines two parallel longitudinal recesses (6,7) each of said recesses (6,7) receiving a respective one of said rods therein; said means (4,5) for clamping includes a slide (4,5) for each of said recesses (6,7), each slide (4,5) being longitudinally slidably mounted in a respective one of said recesses (6,7) and becoming clamped on said respective one of said rods (2,3) at the end of travel of said slide (4,5) along said respective one of said recesses (6,7).

6. Instrumentation according to claim 5, wherein each slide (4,5) is provided with inclined lateral flanges (17,18,19,20), complementary lateral grooves (15a,16a,15b,16b) are provided in the respective recess (6,7), said inclined lateral flanges (17,18,19,20) being engageable in said complementary lateral grooves (15a,16a,15b,16b) inclined clamping ramps are provided in said grooves, said flanges (17,18,19,20) being co-operative with said clamping ramps, having an angle of inclination of substantially 2° to 6° relative to the axis of the respective rod (2,3).

7. Instrumentation according to claim 6, wherein each flange (17,18,19,20) of each slide (4,5) has an end which is the first to enter the respective recess (6,7) and is chamfered and said respective recess (6,7) has an entrance which is chamfered whereby the insertion of said slides (4,5) in said recesses (6,7) is guided.

8. Instrumentation according to any of claims 5 to 7, comprising semi-cylindrical channels (13,14,21,22) forming seats for said rods (2,3) provided in said recesses (6,7) of said blocks (1) and in said slides (4,5).

9. Instrumentation according to any of claims 5 to 8, further comprising a bore (9,11) defined in the bottom of a respective one of each of said recesses (6,7) and a bone anchorage screw (8,10) inserted in each of said bores (9,11).

10. Instrumentation according to claim 9, wherein said bone anchorage screws (8,10)are supported in a respective one of said bores (8,10) to subtend an angle between said bore anchorage screws for each of said blocks (1) said angle being substantially 25 degrees.

11. Instrumentation according to claim 9 or 10, comprising semi-cylindrical channels (13,14,21,22) forming seats for said rods (2,3) in said recesses (6,7) of said blocks (1) and in said slides (4,5) each bore (9,11) opening onto the respective rod (2,3) so that each bone anchorage screw (8,10) is trapped by a complete engagement of the respective slide (4,5) in the respective recess (6,7).

12. Instrumentation according to any of claims 5 to 11, wherein each slide (4,5) has a surface which is flush with a convex exterior surface (S1,S2) of the respective block (1) so that said convex exterior surface and said surface of each slide (4,5) are together smooth and devoid of any projection.

13. Instrumentation according to any of claims 5 to 12, wherein each block (1) comprises complementary clamping means for the respective slide (4,5)

14. Instrumentation according to claim 13, wherein said complementary clamping means comprise, for each slide (4,5), a screw (23,24) insertable in an opening (25,26) provided in the central part of the respective block (1) between said recesses (6,7), said opening (25,26) communicating through a lateral passage (27) with the adjacent groove (16a,16b) of a lateral flange (18,19) of said slide (4,5), and said screw (23,24) being provided with a conical portion (29) positioned to engage in said lateral passage (27) and come to bear against said flange (18,19) of said slide (4,5) to reinforce the clamping thereof in said groove (16a,16b).

15. Instrumentation according to claim 14, wherein said clamping screw (23,24) comprises, from one end to the other thereof, a screw threaded anchoring stem (28), a conical portion (29) for clamping said slide (4,5) which tapers toward said screw threaded stem (28), a fracture conical portion (31) which tapers toward said clamping conical portion (29) and a screwing head (32).

16. Instrumentation according to any preceding claim, wherein said block (1) is curved in such manner as to have in cross-section a substantially S-shape having one half which constitutes an anterior part and a second half constituting a posterior part, said anterior part having a thickness which is less than the thickness of said posterior part and being capable of being inserted in a cavity provided for this purpose in the vertebral body.

17. Instrumentation according to any preceding claim, wherein one rod (3) is suited to be employed as an anterior rod and has a diameter less than the diameter of the other rod (2) which is suited to be employed as a posterior rod.

18. Instrumentation according to claim 6, wherein the inclinations of said lateral flanges (17,18,19,20) of said slides (4,5) and the inclinations of the associated clamping ramps are inverted between the two slides (4,5) of each block (1).

19. Instrumentation according to claim 5, further comprising a cavity (55) in a wall of each recess (6,7) configured to receive a boring tool therethrough for boring in the vertebral body and inserting an anchorage screw (8,10) therein.

20. Instrumentation according to any preceding claim, comprising an opening (34) in an anterior part of each block (1) for receiving a distraction-compression tool (36).

21. Instrumentation according to claim 13, wherein said complementary clamping means comprise a central aperture (25,26) in each block (1), a screw (23,24) insertable in said central aperture (25,26), said aperture and said screw (23,24) having a sufficient diameter to enable said aperture (25,26) to communicate through lateral passages (27) with adjacent grooves (15a,16a,15b,16b) in lateral flanges (17,18,19,20) of the respective slide (4,5), and said screw (23,24) is provided with a conical portion (29) positioned to be engaged in said lateral passages (27) and come to bear against said flanges (17,18,19,20) of the slide (4,5) and reinforce the clamping thereof in the respective groove (15a,16a,15b,16b).

22. Instrumentation according to claim 6, wherein the inclinations of said ramps and flanges (17,18,19,20) of said slides (4,5) are oriented in the same direction for the slides of the same rod (2,3) and in the opposite direction for the ramps and flanges of the slides (4,5) of the other rod (2,3).

23. Instrumentation according to any preceding claim, wherein said openings in said blocks (1) open onto an exterior surface of said blocks (1) and are adapted to receive said rods (2,3) in a parallel arrangement.

## Patentansprüche

1. Spinal-Osteosynthese-Instrumentarium für eine Herangehensweise von vorn, wobei das Instrumentarium in Kombination folgendes aufweist: zwei starre Stäbe (2, 3), wenigstens zwei Blöcke (1) zur Querverbindung der Stäbe (2, 3) untereinander, wobei die Blöcke (1) Öffnungen definieren, um darin jeweils einen der Stäbe auf eine solche Weise aufzunehmen, daß die Stäbe (2, 3) in den Blöcken (1) in Axialrichtung gleiten und sich um sich selbst drehen können, Mittel (4, 5) zum Einspannen der Stäbe (2, 3) gegen eine Drehung und Translationsbewegung in den Blöcken (1) und Knochenverankerungselemente (8, 10), die mit den Blöcken (1) kombiniert sind, **dadurch gekennzeichnet, daß** die Mittel (4, 5) zum Einspannen der Stäbe (2, 3) gleitfähig innerhalb der Blöcke (1) angeordnet sind und an den Stäben anliegen.

2. Instrumentarium nach Anspruch 1, bei dem die Blöcke (1) dafür geeignet sind, die Stäbe (2, 3) in einer parallelen Anordnung aufzunehmen.

3. Instrumentarium nach Anspruch 1, bei dem jeder Block (1) einen vorderen Teil und einen hinteren Teil und in der Querrichtung von dem vorderen Teil zu dem hinteren Teil eine allgemein gebogene Form hat, die der antero-lateralen Anatomie der Vertebralkörper entspricht, wobei der Block (1) eine Innenfläche (S3) für die Anwendung an einem Vertebralkörper und die folglich konkav ist und eine konvexe Außenfläche (S1, S2) hat.

4. Instrumentarium nach Anspruch 3, bei dem der vordere Teil eine Stärke hat, die geringer als die Stärke des hinteren Teils ist, und einen abgerundeten Rand hat.

5. Instrumentarium nach einem der vorhergehenden Ansprüche, bei dem jeder Block (1) zwei parallele Längsaussparungen (6, 7) definiert, wobei in jeder der Aussparungen (6, 7) jeweils einer der Stäbe aufgenommen wird; die Mittel (4, 5) zum Einspannen einen Schieber (4, 5) für jede der Aussparungen (6, 7) einschließen, wobei jeder Schieber (4, 5) in Längsrichtung gleitfähig in jeweils einer der Aussparungen (6, 7) angebracht ist und am Ende der Bewegung des Schiebers (4, 5) längs der jeweils einen der Aussparungen (6, 7) an dem jeweils einen der Stäbe (2, 3) eingespannt wird.

6. Instrumentarium nach Anspruch 5, bei dem jeder Schieber (4, 5) mit geneigten seitlichen Flanschen (17, 18, 19, 20) versehen ist, in der entsprechenden Aussparung (6, 7) komplementäre seitliche Rillen (15a, 16a, 15b, 16b) bereitgestellt werden, wobei die geneigten seitlichen Flansche (17, 18, 19, 20) mit den komplementären seitlichen Rillen (15a, 16a, 15b, 16b) ineinandergreifen können, wobei in den Rillen geneigte Einspannrampen bereitgestellt werden, wobei die Flansche (17, 18, 19, 20) mit den Einspannrampen zusammenwirken, die einen Neigungswinkel von im wesentlichen 2° bis 6° im Verhältnis zur Achse des entsprechenden Stabes (2, 3) haben.

7. Instrumentarium nach Anspruch 6, bei dem jeder Flansch (17, 18, 19, 20) jedes Schiebers (4, 5) ein Ende hat, das als erstes in die entsprechende Aussparung (6, 7) eintritt und abgeschrägt ist, und die entsprechende Aussparung (6, 7) einen Einlaß hat, der abgeschrägt ist, wodurch das Einsetzen der Schieber (4, 5) in die Aussparungen (6, 7) geführt wird.

8. Instrumentarium nach einem der Ansprüche 5 bis 7, das halbzylindrische Auskehlungen (13, 14, 21, 22) aufweist, die Auflagen für die Stäbe (2, 3) bilden und die in den Aussparungen (6, 7) der Blöcke (1) und in den Schiebern (4, 5) bereitgestellt werden.

9. Instrumentarium nach einem der Ansprüche 5 bis 8, das außerdem eine Bohrung (9, 11), die im Boden der jeweils einen der Aussparungen (6, 7) definiert wird, und eine Knochenverankerungsschraube (8, 10) aufweist, die in jede der Bohrungen (9, 11) eingesetzt wird.

10. Instrumentarium nach Anspruch 9, bei der die Knochenverankerungsschrauben (8, 10) in jeweils einer der Bohrungen (9, 11) gehalten werden, um einen Winkel zwischen den Knochenverankerungsschrauben für jeden der Blöcke (1) zu bilden, wobei der Winkel im wesentlichen 25° beträgt.

11. Instrumentarium nach Anspruch 9 oder 10, das halbzylindrische Auskehlungen (13, 14, 21, 22) aufweist, die Auflagen für die Stäbe (2, 3) in den Aussparungen (6, 7) der Blöcke (1) und in den Schiebern (4, 5) bilden, wobei sich jede Bohrung (9, 11) zu dem entsprechenden Stab (2, 3) öffnet, so daß jede Knochenverankerungsschraube (8, 10) durch einen vollständigen Eingriff des entsprechenden Schiebers (4, 5) mit der entsprechenden Aussparung (6, 7) eingeschlossen wird.

12. Instrumentarium nach einem der Ansprüche 5 bis 11, bei dem jeder Schieber (4, 5) eine Oberfläche hat, die bündig mit einer konvexen Außenfläche (S1, S2) des entsprechenden Blocks (1) ist, so daß die konvexe Außenfläche und die Oberfläche jedes Schiebers (4, 5) zusammen gleichmäßig und ohne jeden Vorsprung sind.

13. Instrumentarium nach einem der Ansprüche 5 bis 12, bei dem jeder Block (1) komplementäre Einspannmittel für den entsprechenden Schieber (4, 5) aufweist.

14. Instrumentarium nach Anspruch 13, bei dem die komplementären Einspannmittel für jeden Schieber (4, 5) eine Schraube (23, 24) aufweisen, die in eine Öffnung (25, 26) eingesetzt werden kann, die im Mittelteil des entsprechenden Blocks (1) zwischen den Aussparungen (6, 7) bereitgestellt wird, wobei die Öffnung (25, 26) durch einen seitlichen Durchgang (27) mit der angrenzenden Rille (16a, 16b) eines seitlichen Flanschs (18, 19) des Schiebers (4, 5) in Verbindung steht und wobei die Schraube (23, 24) mit einem konischen Abschnitt (29) versehen ist, der dafür positioniert ist, in den seitlichen Durchgang (27) einzugreifen und an dem Flansch (18, 19) des Schiebers (4, 5) zu liegen zu kommen, um dessen Einspannung in der Rille (16a, 16b) zu verstärken.

15. Instrumentarium nach Anspruch 14, bei dem die Spannschraube (23, 24) von einem Ende zu deren anderem Ende einen mit Schraubgewinde versehenen Verankerungsschaft (28), einen konischen Abschnitt (29) zum Einspannen des Schiebers (4, 5), der sich zu dem mit Schraubgewinde versehenen Schaft (28) hin verjüngt, einen Bruchkonusabschnitt (31), der sich zu dem konischen Einspannabschnitt (29) hin verjüngt, und einen Schraubenkopf (32) umfaßt.

16. Instrumentarium nach einem der vorhergehenden Ansprüche, bei dem der Block (1) auf eine solche Weise gebogen ist, daß er im Querschnitt im wesentlichen eine S-Form mit einer Hälfte, die einen vorderen Teil darstellt, und einer zweiten Hälfte hat, die einen hinteren Teil darstellt, wobei der vordere Teil eine Stärke hat, die geringer als die Stärke des hinteren Teils ist, und dafür geeignet ist, in einen Hohlraum eingesetzt zu werden, der zu diesem Zweck im Vertebralkörper bereitgestellt wird.

17. Instrumentarium nach einem der vorhergehenden Ansprüche, bei dem ein Stab (3) dafür geeignet ist, als ein vorderer Stab eingesetzt zu werden und einen Durchmesser hat, der kleiner als der Durchmesser des anderen Stabes (2) ist, der dafür geeignet ist, als ein hinterer Stab eingesetzt zu werden.

18. Instrumentarium nach Anspruch 6, bei dem die Neigungen der seitlichen Flansche (17, 18, 19, 20) der Schieber (4, 5) und die Neigungen der dazugehörigen Einspannrampen zwischen den zwei Schiebern (4, 5) jedes Blocks (1) umgekehrt sind.

19. Instrumentarium nach Anspruch 5, das außerdem einen Hohlraum (55) in einer Wand jeder Aussparung (6, 7) aufweist, der für die Aufnahme eines durchführenden Bohrwerkzeugs geeignet ist, um in dem Vertebralkörper zu bohren und darin eine Verankerungsschraube (8, 10) einzusetzen.

20. Instrumentarium nach einem der vorhergehenden Ansprüche, das eine Öffnung (34) in einem vorderen Teil jedes Blocks (1) für die Aufnahme eines Destraktion-Kompressionswerkzeugs (36) aufweist.

21. Instrumentarium nach Anspruch 13, bei dem die komplementären Einspannmittel folgendes aufweisen: eine Mittelöffnung (25, 26) in jedem Block (1), eine Schraube (23, 24), die in die Mittelöffnung (25, 26) eingesetzt werden kann, wobei die Öffnung und die Schraube (23, 24) einen ausreichenden Durchmesser haben, um über die seitlichen Durchgänge (27) die Verbindung der Öffnung (25, 26) mit den angrenzenden Rillen (15a, 16a, 15b, 16b) in den seitlichen Flanschen (17, 18, 19, 20) des entsprechenden Schiebers (4, 5) zu ermöglichen, und die Schraube (23, 24) mit einem konischen Abschnitt (29) versehen ist, der dafür positioniert ist, in die seitlichen Durchgänge (27) einzugreifen und an den Flanschen (17, 18, 19, 20) des Schiebers (4, 5) zu liegen zu kommen, um deren Einspannung in der entsprechenden Rille (15a, 16a, 15b, 16b) zu verstärken.

22. Instrumentarium nach Anspruch 6, bei dem die Neigungen der Rampen und der Flansche (17, 18, 19, 20) der Schieber (4, 5) für die Schieber desselben Stabes (2, 3) in derselben Richtung und in der entgegengesetzten Richtung für die Rampen und Flansche der Schieber (4, 5) des anderen Stabes (2, 3) ausgerichtet sind.

23. Instrumentarium nach einem der vorhergehenden Ansprüche, bei dem sich die Öffnungen in den Blöcken (1) auf eine Außenfläche der Blöcke (1) öffnen und dafür geeignet sind, die Stäbe (2, 3) in einer parallelen Anordnung aufzunehmen.

## Revendications

1. Instrument d'ostéosynthèse vertébrale pour une approche antérieure, ledit instrument comprenant en combinaison: deux tiges rigides (2, 3), au moins deux blocs (1) destinés à interconnecter transversalement lesdites tiges (2, 3), lesdits blocs (1) définissant des ouvertures pour recevoir respectivement une desdites tiges, de sorte que lesdites tiges (2, 3) peuvent être glissées axialement et par rotation autour d'elles-mêmes dans lesdits blocs (1), des moyens (4, 5) pour fixer lesdites tiges (2,3) contre une rotation et une translation dans lesdits blocs (1), et des éléments d'ancrage dans l'os (8, 10) combinés avec lesdits blocs (1), **caractérisé en ce que** lesdits moyens (4, 5) destinés à fixer lesdites tiges (2, 3) sont agencés par glissement dans lesdits blocs (1) et s'appuient contre lesdites tiges.

2. Instrument selon la revendication 1, dans lequel lesdits blocs (1) sont destinés à recevoir lesdites tiges (2, 3) dans un agencement parallèle.

3. Instrument selon la revendication 1, dans lequel chaque bloc (1) comporte une partie antérieure et une partie postérieure et a une forme généralement courbée dans une direction transversale de ladite partie antérieure vers ladite partie postérieure, correspondant à l'anatomie antéro-latérale des corps vertébraux, ledit bloc (1) définissant une surface interne (S3) en vue de l'application sur un corps vertébral et ayant donc une forme concave, et une surface externe convexe (SI, S2).

4. Instrument selon la revendication 3, dans lequel ladite partie antérieure a une épaisseur inférieure à l'épaisseur de ladite partie postérieure et comporte un bord arrondi.

5. Instrument selon l'une quelconque des revendications précédentes, dans lequel chaque bloc (1) définit deux évidements longitudinaux parallèles (6, 7), chacun desdits évidements (6, 7) recevant une desdites tiges respectives; ledit moyen (4,5) de fixation englobant un coulisseau (4, 5) pour chacun desdits évidements (6, 7), chaque coulisseau (4, 5) étant monté longitudinalement par glissement dans un desdits évidements respectifs (6, 7) et étant fixé sur une desdites tiges respectives (2,3) à la fin du déplacement dudit coulisseau (4, 5) le long d'un desdits évidements respectifs (6, 7).

6. Instrument selon la revendication 5, dans lequel chaque coulisseau (4, 5) comporte des brides latérales inclinées (17, 18, 19, 20), des rainures latérales complémentaires (15a, 16a, 15b, 16b) étant agencées dans l'évidement respectif (6, 7), lesdites brides latérales inclinées (17, 18, 19, 20) pouvant s'engager dans lesdites rainures latérales complémentaires (15a, 16a, 15b, 16b), des rampes de serrage inclinées étant agencées dans lesdites rainures, lesdites brides (17, 18, 19, 20) coopérant avec lesdites rampes de serrage, et formant un angle d'inclinaison compris pratiquement entre 2° et 6° par rapport à l'axe de la tige respective (2, 3).

7. Instrument selon la revendication 6, dans lequel chaque bride (17, 18, 19, 20) de chaque coulisseau (4, 5) comporte une extrémité rentrant en premier lieu dans l'évidement respectif (6, 7) et étant chanfreinée, ledit évidement respectif (6, 7) comportant une entrée chanfreinée, l'insertion desdits coulisseaux (4, 5) dans lesdits évidements (6, 7) étant ainsi guidée.

8. Instrument selon l'une quelconque des revendications 5 à 7, comprenant des canaux semi-cylindriques (13, 14, 21, 22) formant des sièges pour lesdites tiges (2, 3) agencés dans lesdits évidements (6, 7) desdits blocs (1) et dans lesdits coulisseaux (4, 5).

9. Instrument selon l'une quelconque des revendications 5 à 8, comprenant en outre un alésage (9, 11) défini dans le fond dans un évidement respectif de chacun desdits évidements (6, 7) et une vis d'ancrage dans l'os (8, 10) insérée dans chacun desdits alésages (9,11).

10. Instrument selon la revendication 9, dans lequel lesdites vis d'ancrage dans l'os (8, 10) sont supportées dans un desdits alésages respectifs (9, 11) pour former un angle entre lesdites vis d'ancrage dans l'os pour chacun desdits blocs (1), ledit angle correspondant pratiquement à 25 degrés.

11. Instrument selon les revendications 9 ou 10, comprenant des canaux semi-cylindriques (13, 14, 21, 22) formant des sièges pour lesdites tiges (2, 3) dans lesdits évidements (6, 7) desdits blocs (1), et dans lesdits coulisseaux (4, 5), chaque alésage (9, 11) s'ouvrant sur la tige respective (2, 3), de sorte que chaque vis d'ancrage dans l'os (8, 10) est piégée par un engagement complet du coulisseau respectif (4, 5) dans l'évidement respectif (6, 7).

12. Instrument selon l'une quelconque des revendications 5 à 11, dans lequel chaque coulisseau (4, 5) comporte une surface affleurant une surface externe convexe (S 1, S2) du bloc respectif (1), de sorte que ladite surface externe convexe et ladite surface de chaque coulisseau (4, 5) sont toutes les deux lisses et exemptes de saillies.

13. Instrument selon l'une quelconque des revendications 5 à 12, dans lequel chaque bloc (1) comprend un moyen de fixation complémentaire du coulisseau respectif (4, 5).

14. Instrument selon la revendication 13, dans lequel lesdits moyens de fixation complémentaires comprennent, pour chaque coulisseau (4, 5), une vis (23, 24) pouvant être insérée dans une ouverture (25, 26) agencée dans la partie centrale du bloc respectif (1) entre lesdits évidements (6, 7), ladite ouverture (25, 26) communiquant à travers un passage latéral (27) avec la rainure adjacente (16a, 16b) d'une bride latérale (18, 19) dudit coulisseau (4, 5), ladite vis (23, 24) comportant une partie conique (29) positionnée de sorte à s'engager dans ledit passage latéral (27) et à s'appuyer contre ladite bride (18, 19) dudit coulisseau (4, 5) pour renforcer la fixation correspondante dans ladite rainure (16a, 16b).

15. Instrument selon la revendication 14, dans lequel ladite vis de fixation (23, 24) comprend, d'une extrémité vers l'autre, une queue d'ancrage à filet de vis (28), une partie conique (29) pour fixer ledit coulisseau (4, 5) effilé en direction de ladite queue à filet de vis (28), une partie conique de fracture (31) effilée en direction de ladite partie conique de fixation (29) et une tête de vissage (32).

16. Instrument selon l'une quelconque des revendications précédentes, dans lequel ledit bloc (1) est courbé de sorte à avoir une forme de section transversale pratiquement en S, une moitié constituant une partie antérieure et une deuxième partie constituant une partie postérieure, ladite partie antérieure ayant une épaisseur inférieure à l'épaisseur de ladite partie postérieure et pouvant être insérée dans une cavité correspondante dans le corps vertébral.

17. Instrument selon l'une quelconque des revendications précédentes, dans lequel une tige (3) est destinée à être utilisée comme tige antérieure et a un diamètre inférieur au diamètre de l'autre tige (2), destinée à être utilisée comme tige postérieure.

18. Instrument selon la revendication 6, dans lequel les inclinaisons desdites brides latérales (17, 18, 19, 20) desdits coulisseaux (4, 5) et les inclinaisons des rampes de fixation associées sont inversées entre les deux coulisseaux (4, 5) de chaque bloc (1).

19. Instrument selon la revendication 5, comprenant en outre une cavité (55) dans une paroi de chaque évidement (6, 7) configurée de sorte à recevoir un outil d'alésage pour aléser le corps vertébral et y insérer une vis d'ancrage (8, 10).

20. Instrument selon l'une quelconque des revendications précédentes, comprenant une ouverture (34) dans une partie antérieure de chaque bloc (1) pour recevoir un outil de détraction et de compression (36).

21. Instrument selon la revendication 13, dans lequel lesdits moyens de fixation complémentaires comprennent une ouverture centrale (25, 26) dans chaque bloc (1), une vis (23, 24) pouvant être insérée dans ladite ouverture centrale (25, 26), ladite ouverture et ladite vis (23, 24) ayant un diamètre suffisant pour permettre la communication de ladite ouverture (25, 26) à travers les passages latéraux (27) avec des rainures adjacentes (15a, 16a, 15b, 16b) dans les brides latérales (17, 18, 19, 20) du coulisseau respectif (4, 5), ladite vis (23, 24) comportant une partie conique (29) positionnée de sorte à s'engager dans lesdits passages latéraux (27) et à s'appuyer contre lesdites brides (17, 18, 19, 20) du coulisseau (4, 5) pour renforcer la fixation correspondante dans la rainure respective (15a, 16a, 15b, 16b).

22. Instrument selon la revendication 6, dans lequel les inclinaisons desdites rampes et brides (17, 18, 19, 20) desdits coulisseaux (4, 5) sont orientées dans la même direction pour les coulisseaux de la même tige (2, 3) et dans la direction opposée pour les rampes et brides des coulisseaux (4, 5) de l'autre tige (2, 3).

23. Instrument selon l'une quelconque des revendications précédentes, dans lequel lesdites ouvertures dans lesdits blocs (1) sont ouvertes sur une surface externe desdits blocs (1) et sont destinées à recevoir lesdites tiges (2, 3) dans un agencement parallèle.
